(19) Europäisches Patentamt / European Patent Office / Office européen des brevets

(11) **EP 2 482 797 B1**

(12) **EUROPEAN PATENT SPECIFICATION**

(45) Date of publication and mention
of the grant of the patent:
**12.09.2018 Bulletin 2018/37**

(51) Int Cl.:
*A61K 9/00* *(2006.01)*     *A61M 15/00* *(2006.01)*
*B65D 83/14* *(2006.01)*

(21) Application number: **10757436.0**

(22) Date of filing: **28.09.2010**

(86) International application number:
**PCT/EP2010/064381**

(87) International publication number:
**WO 2011/039196 (07.04.2011 Gazette 2011/14)**

(54) **IMPROVEMENTS TO PRESSURISED METERED DOSE INHALERS**

VERBESSERTE DRUCKINHALATOREN MIT ABGEMESSENER DOSIS

AMÉLIORATIONS APPORTÉES À DES INHALATEURS-DOSEURS PRESSURISÉS

(84) Designated Contracting States:
**AL AT BE BG CH CY CZ DE DK EE ES FI FR GB
GR HR HU IE IS IT LI LT LU LV MC MK MT NL NO
PL PT RO SE SI SK SM TR**

(30) Priority: **29.09.2009 US 246611 P**

(43) Date of publication of application:
**08.08.2012 Bulletin 2012/32**

(73) Proprietor: **Glaxo Group Limited
Middlesex TW8 9GS (GB)**

(72) Inventors:
• **TRILL, Helen Mary
Ware Hertfordshire SG12 0DP (GB)**
• **SPANO, William Fredrick
Auburn, Alabama 36832 (US)**

(74) Representative: **Rice, Jason Neale
GlaxoSmithKline
Global Patents (CN 925.1)
980 Great West Road
Brentford, Middlesex TW8 9GS (GB)**

(56) References cited:
**WO-A1-02/30498     WO-A1-97/32663
WO-A1-03/024623     WO-A1-03/097140
WO-A2-01/87731     WO-A2-02/30499
US-A1- 2008 279 788**

**Description**

Field of the Invention

The present invention relates to a pressurised metered dose inhaler (pMDI) and, in particular, componentry therefor.

Background of the Invention

A pMDI is an inhalation device for delivering a drug to the lung of a patient, typically for topical treatment of respiratory disease, such as asthma and chronic obstructive pulmonary disease (COPD), although also for systemic delivery of a drug.

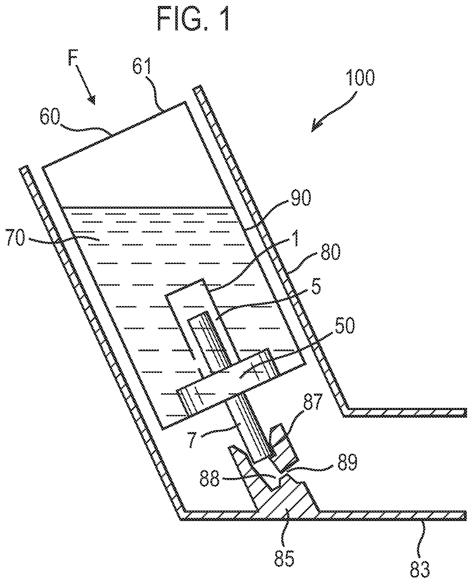

A pMDI comprises a closed container system and an actuator therefor. Figure 1 is a schematic sectional view of a well-known example of a pMDI 100 comprising a hollow, typically plastics, actuator 80 and closed container system 90 mounted for movement in the actuator 80.

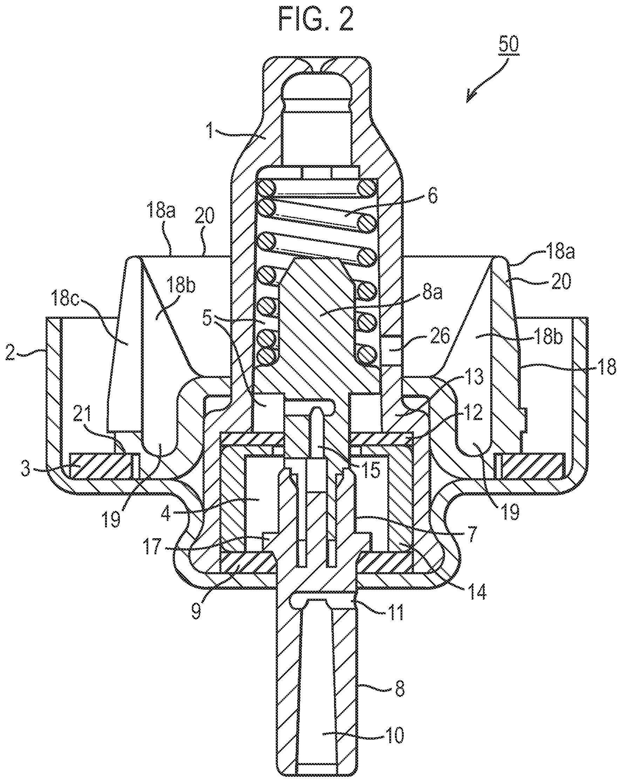

The closed container system of a pMDI comprises a canister (e.g. 60, Fig. 1), typically made from a metal such as aluminium, and a metering valve assembly (e.g. 50, Figs. 1 and 2) which sealingly caps the open end of the canister, e.g. by crimping.

In use, the canister of the closed container system contains a pressurised inhalation drug formulation (e.g. 70, Fig. 1). The formulation comprises the drug and a fluid propellant, and optionally one or more excipients and/or adjuvants. The drug is typically either in solution or suspension in the formulation. Nowadays, the propellant is typically a CFC-free propellant, suitably in liquefied gas form, of which hydrofluoroalkanes (HFA), especially 1,1,1,2-tetrafluoroethane (HFA-134a) and 1,1,1,2,3,3,3-heptafluoropropane (HFA-227), are the most well established for pMDIs.

The function of the metering valve assembly is to discharge a dose of the drug from the closed container system on demand. To this end, the metering valve assembly typically includes a hollow dispensing member or valve stem (e.g. 7, Fig. 1) which is mounted for sliding movement relative to the canister between an extended position, to which the dispensing member is biased by a biasing mechanism (e.g. return spring 6, Fig. 2) in the valve assembly, and a depressed position. Movement of the dispensing member from the extended position to the depressed position results in a dose of the drug being dispensed from the canister through the dispensing member.

Typically, the metering valve assembly is provided with a metering chamber of defined volume. In the extended position of the dispensing member, the bulk pressurised drug formulation in the canister is placed in fluid communication with the metering chamber through the dispensing member so that the metering chamber is filled with pressurised drug formulation. When the dispensing member is depressed, the pressurised drug formulation in the metering chamber is isolated from the canister and placed in fluid communication with the external environment through the dispensing member. Thus, the volume of pressurised formulation in the metering chamber (which includes a metered amount of the drug) is discharged to the external environment via the dispensing member. As the metered volume of the drug suspension/solution is forced from the metering chamber through the valve stem by the high vapour pressure of the propellant, the propellant rapidly vaporises leaving a fast moving cloud of drug particles (the emitted drug dose).

To ensure that the metering chamber is refilled with the correct amount of the drug substance, in particular for suspension formulations, the pMDI is shaken to homogenise the distribution of the drug in the bulk formulation before the pMDI is actuated to dispense the pending drug dose in the metering chamber.

Metering valve assemblies of the type described are well known in the art and can be obtained from *inter alia* Bespak Plc (King's Lynn, Norfolk, United Kingdom), Valois S.A.S. (Le Neubourg, France) and others companies known in the art. Other forms of metering valve assemblies are also known in the art.

It is known to coat the internal surfaces of the canister and/or metering valve assembly which come into contact with the drug formulation with a non-stick material, e.g. a fluoropolymer, to inhibit the drug adhering to the internal surfaces. As an example, it is known to coat the inside surfaces of the canister with a blend of polytetrafluoroethylene (PTFE) and polyethersulphone (PES), for instance as described in US patent No. 6,131,566. It is also known to coat the metering chamber of the metering valve assembly with a fluoropolymer layer, such as of perfluorohexane, for instance by cold plasma polymerisation, as disclosed in WO-A-99/42154. However, in the specific Examples of the invention hereinafter to described, the metering valve assembly is not coated, although use of such a coating is within the scope of the invention.

The actuator of a pMDI has a hollow construction so that the closed container system is able to the mounted therein, such as shown in Figure 1. The actuator is generally formed of a plastics material, for instance by moulding. A stem block of the actuator (e.g. 85, Fig. 1) receives the dispensing member of the closed container system. The stem block has a passageway (e.g. 88, Fig. 1) with an inlet end (e.g. 87, Fig. 1) for receiving the dispensing member and an outlet end (e.g. 89, Fig. 1), which faces a dispensing outlet of the actuator, typically a mouthpiece (e.g. 83, Fig. 1). Ordinarily, as in Figure 1, the stem block holds the dispensing member stationary in the actuator and the canister is moved relative thereto to cause the dispensing member to be displaced from the extended position to the depressed position so that the pressurised volume of the formulation with drug dose in the metering chamber is dispensed out of the dispensing outlet of the actuator via the internal passageway of the stem block. Other actuator arrangements for causing the relative movement of the dispensing member are also known in the art.

In use, a patient in need of a dose of the drug takes the pMDI, with closed container system mounted in the actuator, and concurrently inhales on the dispensing outlet whilst causing the canister to move relative to the dispensing member so as to open the valve assembly and release a metered volume of the drug formulation. The drug particles discharged through the dispensing outlet are entrained into the patient's respiratory tract by their inspiratory airflow.

In the well-known form of pMDI 100 shown in Figure 1, the base 61 of the canister 60 protrudes from, or is in close proximity to, an opening in the actuator 80 and the patient actuates the pMDI 100 by pushing (arrow F, Fig. 1) on the canister base 61 to move the canister 60 relative to the dispensing member 7, which is held stationary in the stem block 85. As a result, the metering valve assembly opens to discharge a metered volume of the drug formulation. The drug formulation is discharged into the stem block conduit 88 and directed through the mouthpiece 89 by the outlet end 89, which acts as a spray orifice. The pMDI 100 is of the type known as a "breath-coordinated" (or "press-and-breathe") pMDI since the patient has to coordinate their inhalation with manual actuation of the closed container system.

In other pMDIs, the actuator is provided with a firing mechanism to cause the relative movement between the dispensing member and the canister upon patient operation of the firing mechanism. In one form, the patient operates a manual trigger member, e.g. lever, of the firing mechanism to manually operate the firing mechanism. In this instance, the firing mechanism may provide mechanical advantage, although the pMDI is still breath-coordinated. However, in another form, the inspiratory airflow produced by the patient itself is the trigger which operates the firing mechanism (a so-called "breath-operated pMDI"). This has the advantage that operation of the pMDI is coordination free.

Typically, pMDIs are hand-held (e.g. portable), such as the pMDI 100 of Figure 1 and those of the general type described in the preceding paragraph.

For a pMDI to be approved for patient use, a pMDI has to meet certain regulatory requirements. One regulatory requirement is that each time the metering valve assembly is operated it meters substantially the same quantity of drug (metered dose); the so-called "dose content uniformity". Moreover, the proportion of each metered dose consisting of drug particles of the correct size (aerodynamic particle diameter) for deposition at the required site in the lungs also needs to be uniform (stable); i.e. within specified limits. This proportion is known as the fine particle mass (FPM), and represents the proportion of the metered dose made up of drug particles with an aerodynamic particle diameter in the range of about 1 micron to about 5 microns. As well-known in the art, the FPM of a pMDI can be determined on a cascade impactor, such as the Anderson Cascade Impactor (ACI). The FPM is that amount of the metered dose collected on particular stages of the cascade impactor (e.g. ACI).

The drug particles of the metered dose not in the FPM are either too large, and deposit in the patient's throat or higher parts of the lung, or too small, and go straight into the blood stream or are expelled from the patient's body on exhalation.

A problem which can be encountered with a pMDI is ingress of moisture into the closed container system, for instance at the seal (e.g. 3, Fig. 2) between the metering valve assembly and the canister, and the adverse effect this has on the stability of the drug formulation in the system, such as the loss of consistency (instability) of the FPM.

A previously proposed solution to this problem is to enclose the pMDI in a moisture impermeable overwrap post-manufacture, for instance as used on MDI products sold in the United States of America (see also US patent No. 6,119,853). The overwrap prevents moisture ingress into the closed container system, but only until the pMDI is removed therefrom, when the patient wishes to first use the pMDI. For the rest of the period when the MDI is being used, no moisture protection is provided.

As additional background art there may be mentioned WO02/30498, WO02/30499 and WO03/024623, each mentioning use of moisture-absorbing means in pMDIs, and WO97/32663 which mentions moisture absorbing desiccant-entrained polymers.

The present invention proposes means to stabilise the FPM of a pMDI.

Summary of the Invention

According to an aspect of the present invention there is provided use of a desiccant-entrained material of the type defined in a pMDI closed container system as set forth in claim 1.

A "desiccant-entrained material of the type defined" is a material which comprises a water permeable polymer body which contains a desiccant to capture water molecules within the body. The desiccant-entrained material is of the type formed by mixing, e.g. blending, the base polymer, desiccant and the channelling agent that facilitates the transmission of water into the body. Such a material is disclosed in US-A-5911937 of Capitol Speciality Plastics, Inc. (CSP), where the channelling agent is blended with the base polymer and the desiccant. This type of material is also sold by CSP Technologies (Auburn, Alabama, USA).

In another aspect of the present invention there is provided a pMDI metering valve for a pMDI closed container system as set forth in claim 12.

In a further aspect of the present invention there is provided a pMDI closed container system as set forth in claim 13. The closed container system may be containing an inhalation drug formulation .

In a yet further aspect of the present invention there is provided a method of stabilising the fine particle mass (FPM) of an inhalation drug formulation emitted by a pMDI closed container system as set forth in claim 14. In an embodiment, the FPM is stable after storage for a period of (i) at least 6 months at a temperature of 40°C and at 75% relative humidity (RH), or (ii) at least 9 months, optionally at least 12 months, at a temperature of 30°C and at 65% RH, as determined with an Anderson Cascade Impactor.

The purpose of the desiccant-entrained material is to stabilise (maintain consistency of) the fine particle mass (FPM) of an inhalation drug formulation emitted by the closed container system by capturing moisture present (whether initially and/or through ingress) in the closed container system which would otherwise be present in the formulation. In embodiments of the invention, the use of the desiccant-entrained material stabilises the FPM without use of a moisture impermeable overwrap for the pMDI, i.e. unprotected. In embodiments of the invention, the FPM is stable after storage unprotected for a period of at least 6 months at a temperature of 40°C and at 75% relative humidity (RH) or for a period of at least 9 months, optionally at least 12 months, at a temperature of 30°C and at 65% RH.

For the US Food and Drug Administration (FDA), the normal storage condition for stability testing of pMDIs is 25°C/60%RH, but accelerated testing is allowed to be carried out at harsher storage conditions. In this regard, stability results obtained for pMDIs after storage for a given time T at 40°C/75%RH are taken to be the results that would be obtained at the normal storage condition at time point 3xT. The storage condition 30°C/65%RH is normal for stability testing pMDIs for many 'rest of world' regulatory authorities.

As previously described, the FPM is that fraction of the drug dose delivered from the system whose particles have an aerodynamic particle diameter to enable them to deposit at the target site in a patient's lungs. In other words, the desired effect of the inhalation drug formulation is provided by the FPM of the delivered dose. Typically, the drug particles in the FPM have an aerodynamic particle diameter in the range of 1-5 microns. The FPM delivered by a MDI closed container system is able to be determined by testing with an Anderson Cascade Impactor (ACI), the FPM corresponding to the fraction of drug particles collected on stages 3-5 (inclusive) of the ACI for US regulatory submissions (US standard) or stages 2-6 of the ACI for rest of world (RoW) regulatory submissions (RoW standard), as is well known in the art.

It is thought that the FPM for inhalation drug formulations can be destabilised (i.e. less consistent) over the shelf-life by an increase in the moisture content in the formulation, e.g. through moisture ingress into the MDI closed container system and/or moisture present in the MDI closed container system components (e.g. valve parts) transferring into the formulation during the shelf-life.

The FPM delivered by a MDI closed container system has to be consistent for the shelf-life of the system; that is to say, the FPM of each delivered dose has be consistent, e.g. within a defined range, for the shelf-life of the system. This may be determined by a stability test using an ACI with either the US standard or the RoW standard.

Thus, by managing the moisture levels in the formulation, the FPM may be maintained more consistent (i.e. stabilised) from dose-to-dose over the shelf-life of the system.

In embodiments of the invention, after storage of the closed container system at 40°C and 75% relative humidity (RH) for at least 6 months after filling with an inhalation drug formulation, the FPM is stable as determined with an ACI using the US standard. This is equivalent to a stable FPM for an 18 month shelf-life at the normal FDA-approved stability storage condition of 25°C/60%RH as determined on an ACI using the US standard.

In embodiments of the invention, the desiccant-entrained material is comprised in the metering valve of the closed container system. For example, an existing valve component comprises the desiccant-entrained material (i.e. comprised in a replacement valve component) or a component comprising the desiccant-entrained material is added to the valve (i.e. as a new valve accessory). The desiccant-entrained material may be comprised in a valve ring, such as of the general or specific type disclosed in US patent No. 6,170,717, in which case it may be advantageous for the base polymer of the desiccant-entrained material to be nylon, a currently-used material for the ring in approved pMDIs. The valve ring may be lengthened so that more surface area presented by the desiccant-entrained material is in contact with the formulation. Alternatively, or additionally, the desiccant-entrained material may be comprised in a valve component selected from the group consisting of the valve body, the metering chamber, stem seal(s), valve-canister gasket and any combination thereof. Conveniently, the replacement valve component or valve accessory is made from the desiccant-entrained material, e.g. moulded therefrom.

In embodiments of the invention, the desiccant-entrained material is comprised in a component of the closed container system which is in contact with an inhalation drug formulation when present therein.

In embodiments of the invention, the desiccant-entrained material is comprised in a component of the closed container system which is loose inside the canister, for example a lozenge. In embodiments of the invention, to maximise the amount of the outer surface of the loose component that contacts the formulation the loose component is denser than the inhalation drug formulation to be present in the closed container system so as to sink therein. In this way, the maximum amount (if not all) of the loose component will be submerged in the formulation.

In embodiments of the invention, the desiccant-entrained material is comprised in a sleeve, ring or coil located in the canister. The sleeve may have an open mesh or closed construction. The ring may be a split ring (i.e. to give a C-shape in diametrical cross-section (i.e. perpendicular to the ring axis)). The sleeve, ring or coil may be adapted to slide up and down in the canister on shaking thereof, such as to remove drug particles deposited on internal surfaces of the canister.

In embodiments of the invention, the desiccant-entrained material comprises a channelling agent to form a network of water transmitting channels.

The chemical formula of PEG is $H(OCH_2CH_2)_nOH$, where n is the average number of repeating oxyethylene groups

In embodiments of the invention, the PEG of the channelling agent is selected such as not to produce a leachable in an amount at or above the Qualification Threshold (QT) for the particular inhalation drug formulation to be used in the pMDI closed container system.

The use for the channelling agent of PEG of at least 8000 (e.g. PEG with $n \geq 180$, such as at least PEG 10000) is to reduce the likelihood of leaching of the PEG into an inhalation drug formulation when present in the system. In embodiments of the invention the PEG of the channelling agent is of an average molecular weight which is of at least about 16000 (e.g. PEG with $n \geq 360$, such as at least PEG 20000), and optionally greater than 8000 and no more than 350000 (e.g. PEG with $7950 \geq n > 180$, such as greater than PEG 10000 but no more than PEG 300000).

In embodiments of the invention, the channelling agent is present in an amount of up to 15% by mass of the desiccant-entrained material, optionally in the range of 1-10% by mass and further optionally in the range of 3-10% by mass.

When expressing a constituent of the desiccant-entrained material as "% by mass" means the percentage of the total mass of the desiccant-entrained material (i.e. of the total mass of the overall desiccant-entrained material composition) which is made up by that constituent. Typically, this is the starting mass percentage of that constituent in the overall composition used to form the material.

In embodiments of the invention, the base polymer of the desiccant-entrained material is a plastics material, for example a thermoplastics polymer. In embodiments of the invention, the base polymer is acetal, a well-established, well-tolerated plastics material for valve parts. In alternative embodiments of the invention, the base polymer is polypropylene.

In embodiments of the invention, the base polymer is present in the range of 30-70% by mass of the desiccant-entrained material, optionally in the range of 35-60% by mass, yet further optionally in the range of 40-60% by mass and more optionally in the range of 40-50% by mass.

In embodiments of the invention, the desiccant is an adsorbent of water molecules, for instance a molecular sieve. A molecular sieve has the advantage that its uptake of water molecules is a one-way process. A molecular sieve is a material containing tiny pores that are used as an adsorbent. The pore size is such that water molecules are small enough to pass through the pores and be adsorbed. In embodiments of the invention, the molecular sieve has a pore size of 4 Angstroms.

In embodiments of the invention, the desiccant is present in the range of 30-70% by mass of the desiccant-entrained material, optionally in the range of 40-60% by mass, more optionally in the range of 45-60% by mass, further optionally in the range of 45-55% by mass, yet further optionally in the range of 45-50% by mass. The desiccant may also be present in the range of 50-60% by mass.

In embodiments of the invention, the desiccant-entrained material comprises the base polymer in the range of 35-60% by mass, optionally in the range of 40-60%, the desiccant in the range of 40-60% by mass, optionally in the range of 45-60% by mass, and the channelling agent in the range of 1-15% by mass, optionally 3-10% by mass.

In embodiments of the invention, the desiccant-entrained material is a blend of polypropylene, the PEG , and a molecular sieve, optionally with a pore size of 4 Angstroms.

The system may contain an inhalation drug formulation comprising at least one drug and a propellant, optionally with one or more excipients and/or one or more adjuvants. In embodiments of the invention, the formulation consists essentially of the at least one drug and the propellant. The formulation may be a suspension or solution formulation. In embodiments of the invention, the formulation is a suspension formulation.

In embodiments of the invention, the propellant is a CFC-free propellant, typically HFA-134 or HFA-227 or mixtures thereof. In embodiments of the invention, the CFC-free propellant is HFA-134a.

In embodiments of the invention, the at least one drug is for treatment of a respiratory disease, for instance asthma and/or chronic obstructive pulmonary disease (COPD).

In embodiments of the invention, the formulation comprises at least one drug selected from the group consisting of salbutamol or a physiologically acceptable salt thereof, salmeterol or a physiologically acceptable salt thereof (e.g. xinafoate), fluticasone and a fluticasone ester (e.g. fluticasone propionate or fluticasone furoate). In embodiments of the invention, the formulation comprises salmeterol or a salt thereof and a fluticasone ester. In embodiments of the invention, the formulation comprises salmeterol xinafoate and fluticasone propionate. In embodiments of the invention, the at least one drug is in particulate form in the formulation (i.e. a suspension).

In embodiments of the invention, the formulation comprises salbutamol (also known as albuterol) or a physiologically acceptable salt thereof. In embodiments of the invention, the salbutamol salt is salbutamol sulphate. In embodiments of the invention, the formulation comprises salbutamol or a physiologically acceptable salt thereof (e.g. sulphate) and a CFC-free propellant, for instance selected from HFA134a and HFA227 and mixtures thereof. The formulation may consist essentially of salbutamol or a physiologically acceptable salt thereof (e.g. sulphate) and the CFC-free propellant, for instance salbutamol sulphate and HFA134a. In embodiments of the invention, the salbutamol or salt thereof is in particulate form in the formulation.

In embodiments of the invention, the channelling agent is PEG of at least 8000 (e.g. $n \geq 180$, such as at least PEG 10000) and more optionally of at least about 16000 (e.g. PEG with $n \geq 360$, such as at least PEG 20000), and the drug formulation comprises salbutamol sulphate and a CFC-free propellant. The CFC-free propellant may be HFA-134a. The salbutamol sulphate may be a suspension in the formulation. The formulation may consist essentially of the salbutamol sulphate and the propellant:

In embodiments of the invention the FPM testing on the ACI is carried out with the closed container system mounted in a pMDI actuator.

Other aspects, embodiments and features in accordance with the invention are disclosed in the accompanying Examples which follow.

According to the present invention, each aspect thereof can incorporate any one or more features of any one or more of the Examples and/or embodiments and/or other aspects.

Brief Description of the Figures of Drawings

Figure 1 is a schematic sectional view of a pMDI;

Figure 2 is a side sectional view of a metering valve assembly, in an inverted (in use) orientation, as used in the Examples;

Figure 3 shows the FPM and moisture results from Example 1;

Figure 4 is a Liquid Chromatography Mass Spectroscopy (LC-MS) spectrum for PEG extractables from Example 1;

Figure 4A shows a variability plot of PEG leachables from Example 1; Figures 5 to 8 show FPM and moisture level results from Example 2 at different storage conditions;

Figure 9 is a variability plot of PEG leachables from Example 2; and

Figures 10 to 15 show the FPM and moisture level results from Example 3 at different storage conditions.

Examples of the Invention

In Figure 2 there is shown a pMDI metering valve assembly 50 for use in a pMDI, such as of the standard type shown in Figure 1 to which reference is made in the following description. The metering valve assembly is as described in US patent No. 6,170,717. The use of relative terms such as "lower", "upper" and the like are with respect to the inverted, use orientation of the metering valve assembly shown in Figure 1.

The metering valve assembly 50 has a valve body 1 which is formed at its lower part with an annular metering chamber 4, and at its upper part with an annular sampling chamber 5 which also acts as a housing for a return spring 6. Inside the valve body 1 is disposed a valve stem 7, a lower part 8 of which extends outside the valve through an opening in an annular, lower stem seal 9 and a ferrule 2. The lower stem part 8 is formed with an inner, axial or longitudinal canal 10 ending at an opening at the outer end of the valve stem 7 and intersecting with a radial passage 11 at its inner end.

The valve stem 7 further comprises an upper part 8a which passes through the opening in an annular, upper stem seal 12. The valve stem 7 is slidably mounted in the metering valve and the lower and upper stem parts 8, 8a have a diameter such that they respectively sealingly slidably engage the circumferential periphery of the openings of the lower and upper stems seals 9, 12. In other words, a dynamic seal is effected between the valve stem 7 and the stems seals 9, 12.

The upper stem seal 12 is held in position against a step 13 formed in the valve body 1 between its lower and upper parts by a sleeve 14 which defines the metering chamber 4 between the lower stem seal 9 and upper stem seal 12. The metering chamber 4 is not coated *supra*, but may be if desired. The valve stem 7 has a passage 15 which, when the stem is biased by the return spring 6 to its return position, as shown, provides communication between the metering chamber 4 and the sampling chamber 5, which itself communicates with the interior of the canister 60 to which it is mounted, via one or more orifices 26 formed in the side of the valve body 1. The orifice(s) 26 allow the pressurised inhalation drug formulation 70 in the canister 60 to flow freely into the sampling chamber 5.

The valve stem 7 is provided with a shoulder 17 which abuts against the lower stem seal 9 in the return or rest position of the valve stem 7. In the rest position, the radial passage 11 opens below the lower stem seal 9 so that the metering chamber 4 is isolated from the canal 10 and the drug formulation in the metering chamber 4 cannot escape to the exterior environment.

A separately-formed plastics gathering ring 18 is mounted by friction fit to the valve body 1 below the orifice(s) 26. The ring 18 has an outer ring wall 18a, a plurality of radial walls 18b extending inwardly from the outer ring wall 18a, and a U-shape trough segment 19 between each adjacent pair of radial walls 18b.

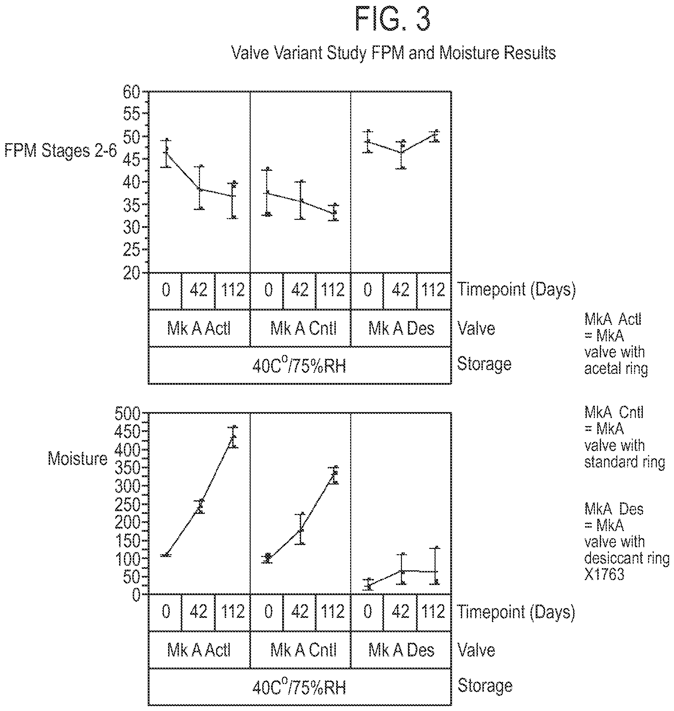

The outer ring wall 18a is formed with a number of equi-angularly spaced slots 18c (only one shown) to divide the outer ring wall 18a into a plurality of vanes 20 (see US patent No. 6,170,717, Figure 3).

The lower part of the ring 18 is further provided with a seat 21 for a gasket 3. The gasket 3 forms the seal between the metering valve assembly 50 and the canister 60 when the metering valve assembly 50 is crimped to the canister through the ferrule 2, as known in the art. The seat 21 helps to locate the gasket 3 in the correct position during assembly and also allows the inner diameter of the gasket 3 to be increased, thereby reducing the mass of the gasket 3 and the gasket surface area exposed to the drug formulation within the canister 60.

To dispense a dose of the drug formulation from the closed container system 90, the user depresses the valve stem 7 inwardly against the force of the return spring 6. In operation of the pMDI 100, this is achieved by the valve stem 7 being held stationary in the stem block 85 of the actuator 80 and the canister 60 being urged towards the stem block 85.

As a result, firstly both openings to the passage 15 come to lie on the upper side of the upper stem seal 12 remote from the metering chamber 4 and then the radial passage 11 moves into the metering chamber 4. Thus, the metered volume of the drug formulation in the metering chamber 4 exits the valve assembly 50 under pressure through the valve stem 7 via the radial passage 11 and the outlet canal 10.

On release of the force F used to depress the valve stem 7, the return spring 6 acts to return the valve stem 7 to the illustrated return position so that the passage 15 once again provides flow communication between the metering chamber 4 and the sampling chamber 5. Accordingly, drug formulation 70 flows into the metering chamber 4 to refill it ready for next actuation of the metering valve assembly 50.

## EXAMPLES

In the following Examples, batches of pMDI closed container systems, including controls and placebos, were manufactured and made-up of sub-batches as detailed herein. The closed container systems consisted of:

- an aluminium canister (Presspart GmbH & Co. KG, Marsberg, Germany), spray coated on the inside surface with a blend of polytetrafluoroethylene (PTFE) and polyethersulphone (PES) (E.I. Du Pont de Nemours and Company, USA) and as further detailed in US patent No. 6,131,566; and
- crimped to the canister, a metering valve assembly (Valois SAS, Le Neubourg, France) having the structure shown and described above with reference to Figure 2, except as otherwise described herein, and a metering chamber with a volume of 63 microlitres.

Except where otherwise stated, each batch was filled with an inhalation drug formulation consisting only of a suspension of salbutamol sulphate (also known as albuterol sulphate) and HFA 134a propellant. pMDIs with this drug formulation are sold by the GlaxoSmithKline group of companies under e.g. the trade name Ventolin™ (hereinafter referred to as Ventolin™ HFA MDI or similar). Each salbutamol sulphate containing batch was manufactured to provide 200 actuations of 100 micrograms/actuation of salbutamol sulphate from the valve (i.e. "ex-valve").

Except if otherwise stated, by "control" is meant a pMDI closed container system filled with the Ventolin™ HFA MDI formulation *supra* but not including any desiccant-entrained material of the type defined, and by "placebo" is meant a pMDI closed container system filled only with HFA134a propellant.

Each batch was then stored unprotected (i.e. not overwrapped *supra*) at different environmental conditions in an inverted orientation (valve down), as is established for regulatory testing of pMDIs. At various time intervals, a number of samples of each sub-batch were removed from storage to determine (i) the FPM by an Anderson Cascade Impactor (ACI) (covered under aerodynamic particle size distribution in Monologue - USP 601), and (ii) the moisture level inside the closed container systems by a Karl Fischer test (Water Determination Monologue - USP 921). Unless stated otherwise, the ACI and moisture testing was carried out using five samples of each sub-batch.

The FPM results reported herein indicate the amount of the drug constituting the FPM (micrograms). In other words, the amount captured on the relevant stages of the ACI. The moisture results reported herein are as parts per million (ppm).

The ACI testing herein was carried out either according to a US standard or to a rest of the world (RoW) standard, as identified herein. As will be understood by the skilled reader, the ACI testing was carried out using the following conditions:

- US standard: a flow rate of 28.3 L/min of air through the ACI, a temperature of 23°C (+/-5°C) and a humidity of 90%RH (+/- 5%RH).

- RoW standard: a flow rate of 28.3 L/min of air through the ACI, a temperature of 20°C (+/-5°C) and a humidity of 50%RH (+/- 5%RH).

The closed container systems were also loaded into a suitable plastics actuator for the ACI tests, of the type shown in Figure 1 hereof (i.e. the emitted doses were delivered into the ACI through the mouthpiece of the actuator).

### Example 1

In this Example, the pMDI closed container systems in the respective batch used a metering valve assembly referred to as the "Mark A" or "Mk A" valve. Unlike the valve shown in Figure 2, the Mk A valve has a valve body 1 with 3 short slots instead of orifices 26, as will be understood by reference to Figure 1 of US patent No. 6,170,717 *supra*. The Mk A valve components are made of the following materials:

| Component (Ref. No. in Fig. 2) | Material |
|---|---|
| Valve body (1) | Acetal |
| Spring (6) | Stainless steel |
| Lower valve stem (8) | Acetal |
| Upper valve stem (8a) | Acetal and PTFE blend |
| Ferrule (2) | Aluminium |
| Sleeve (14) | Acetal |
| Stem seals (9, 12) | EPDM rubber* |
| Gasket (3) | EPDM rubber* |
| Ring (18) | See below |
| * EPDM rubber = ethylene-propylene diene monomer rubber | |

In this Example, there were 3 sub-batches called "Mk A Actl", "MK A Cntl" and "Mk A Des". In the Mk A Cntl sub-batch the ring 18 was made from nylon with six slots 18c. The Mk A Actl sub-batch used an acetal ring 18. The Mk A Des sub-batch used a ring 18 made from a desiccant-entrained material of the type defined and available from CSP Technologies (Auburn, Alabama, USA). The ring 18 in the Mk A Des sub-batch was of a desiccant-entrained material made from a solidified blend of acetal (Ticona Celcon) (35% by mass) as base resin, Ceca 4A* molecular sieve (60% by mass) as desiccant, and polyethylene glycol (PEG) of an average molecular weight in the range of 3600-4400 (5% by mass) as channelling agent.. The channelling agent was PEG 4000, sold by The Dow Chemical Company as Carbowax 4000P. This desiccant-entrained material is referred to as the "X1763 blend". The rings 18 made of acetal and the X1763 blend were not slotted, but a simple solid ring form.

 * The notation "4A" indicates the molecular sieve has a pore size of 4 Ångströms.

The mass percentages of the ingredients of the X1763 blend (and the other desiccant-entrained materials in the Examples herein) are the starting mass percentages for making the blend, but also correspond to the respective mass percentages in the final solidified blend.

The sub-batches were stored inverted, unprotected (i.e. no overwrap *supra*) at 40°C/75%RH for up to 112 days.

The FPM and moisture results are given in Figure 3. In this Example, the FPM performance test was carried out on the ACI and reported as the sum of stages 2-6 inclusive of the ACI (i.e. ACI test at the RoW standard).

The results show that sub-batch Mk A Des exhibited lower moisture and correspondingly high and stable FPM over the 112 days, compared to the two control sub-batches Mk A Cntl and Mk A Actl. Thus, the use of the desiccant-entrained material gave a marked improvement in FPM stability compared to the control cans.

Extraction Study

The raw acetal material used in the X1763 blend was milled using a cryogenic freezer mill, and sieved to achieve a consistent size range of less than 425$\mu$m. The PEG 4000 and molecular sieve materials of the X1763 blend did not require milling as these raw materials were supplied in powder form. Following milling, each of the raw materials were subject to an automated technique of Accelerated Solvent Extraction (ASE). The samples were extracted with the solvent Dichloromethane (DCM). The samples were also extracted in hexane and IsoPropyl Alcohol (IPA). The extracts were analysed by multiple broad spectrum hyphenated techniques to ensure complete evaluation of the extractables profiles. High Performance Liquid Chromatography Mass Spectroscopy (HPLC-MS) including Atmospheric Pressure Chemical Ionisation (APCI), Electrospray Ionisation (ESI), and also Gas Chromatography Mass Spectroscopy (GC-MS) were used. Before analysis, solvents were blown down and the extract re-suspended in solvents suitable for analysis by the aforementioned analytical techniques.

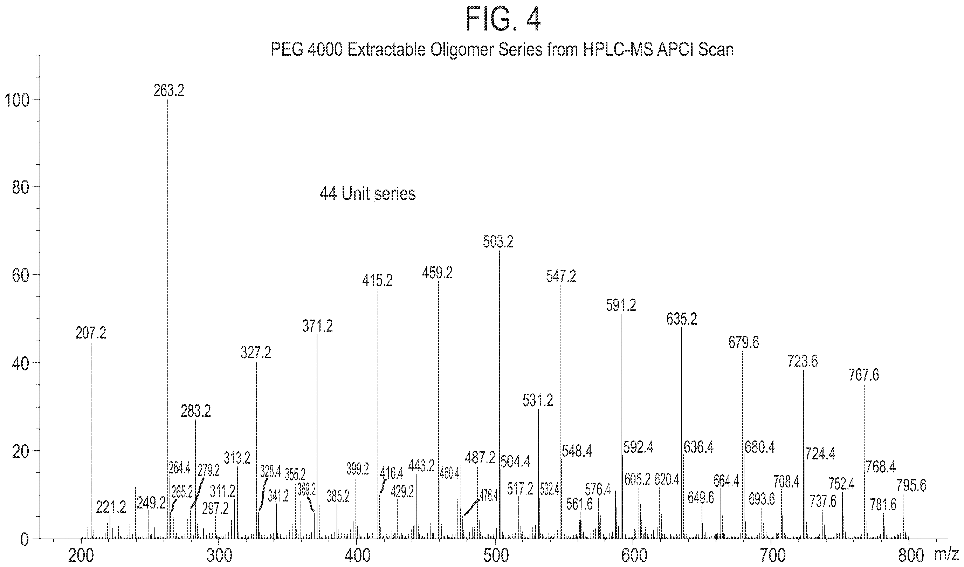

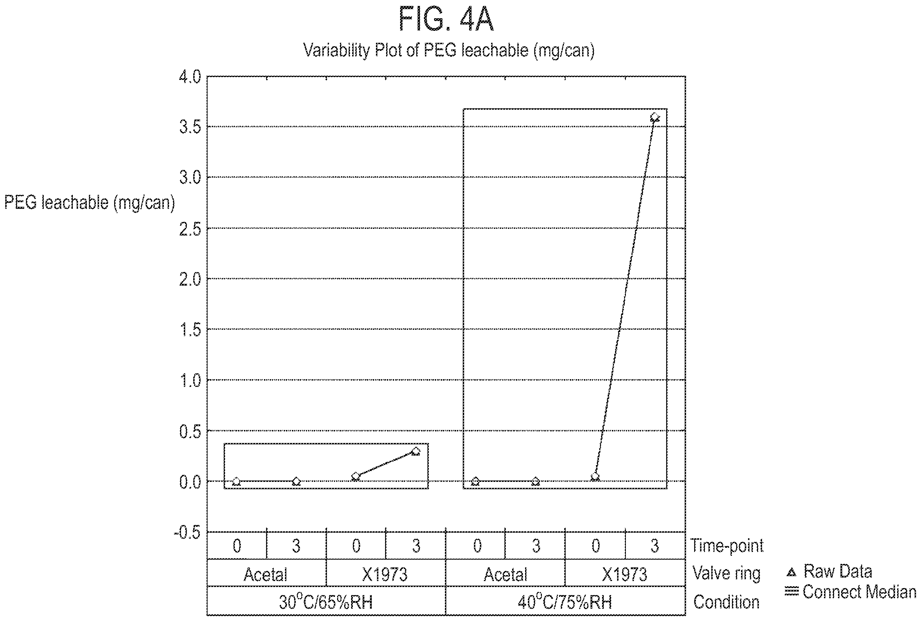

Qualitative analysis by LC-MS APCI and ESI was performed in scan mode using both positive and negative ionisation. The most abundant extractable however was that of PEG 4000 extracts. A number of PEG peaks were found in IPA (7 in total of varying mass ranges), and three PEG peaks in the DCM extract, with characteristic oligomer series of repeating 44 sub-units present in the spectra when analysed by LC-MS (positive ionisation APCI). An example MS spectrum is given in Figure 4.

Identification of PEG 4000

Placebo closed container systems were made up so as to be able to provide 200 actuations of HFA 134a propellant. The placebos were fitted with Mk C valves (details given in Example 2 *infra*) having a simple solid (unslotted) ring 18 made from the X1763 blend. The placebos were put on stability (unprotected/inverted) for 3 months at 40°C/75%RH. After storage, a white powder was found in the placebos once they had been opened and the HFA 134a propellant evaporated. No similar residue was observed in control closed container systems which corresponded to the placebos other than that the ring 18 was a slotted ring made of nylon. Initial identification of this powder was undertaken by Infra-Red (IR) and Time-of-Flight Mass Spectroscopy (QTOF-MS). The IR spectrum of the unknown residue matched that of PEG 4000

Similar retention times and spectral responses were observed by QTOF-MS analysis for the unknown white powder sample and PEG 4000 standard, confirming the material to be PEG 4000, as suspected from the extraction study.

Having identified the powder as a PEG 4000 leachable from the desiccant plastic gathering ring 18, a leachables method was developed using quadropole LC-MS. Ionisation of the PEG as a leachable was achieved using ESI since ESI is suited to higher molecular weight materials as it has a large mass:charge (m/z) ratio range (up to 2500). In order to be sure all of the PEG 4000 material was being detected by the quadropole ESI, it was confirmed by ESI QTOFMS that the material was multiple charging and within range of the detector.

As the PEG 4000 material was present in such high abundance compared to other comparably negligible leachables, a Flow Injection Analysis (FIA) method using an isocratic mobile phase of 1THF:1H2O and Selective Ion Monitoring (SIM) with ESI detection was used to identify the most abundant ions.

It was determined that after 3 months at 40°C/75%RH, 2.7 mg/pMDI closed container system of PEG 4000 had leached into the suspension from the desiccant plastic gathering ring 18 made with the blend of X1763.

In relation to amounts of leachables in a pMDI, the Product Quality Research Institute (PQRI) recommendation to the FDA (8 September 2006) was for a Safety Concern Threshold (SCT) of 0.15$\mu$g per day and a Qualification Threshold (QT) of 5$\mu$g per day for an individual leachable. The SCT is the daily threshold below which a leachable would have a dose so low as to present negligible safety concerns from carcinogenic and non-carcinogenic toxic effects. The QT is the daily threshold below which a given leachable is not considered for safety qualification (toxicological assessments) unless the leachable presents structure activity relationship (SAR) concerns.

As these safety thresholds are expressed in total daily intake (total exposure per day), they must be converted into relative amounts expressed in terms such as amount of an individual leachable in a particular drug product ($\mu$g per pMDI closed container system). This conversion is performed by using information on the drug product such as the total number of actuations of a pMDI, the total number of actuations to be delivered by the pMDI per day, etc which form part of the label claim for a pMDI, as known in the art. The converted SCT is called the Analytical Evaluation Threshold (AET). The AET is defined as the threshold at or above which a particular leachable and/or extractable should be identified and reported for potential toxicological assessment.

The AET for any given pMDI ($\mu$g/pMDI closed container system) can be determined as follows:

$$AET = (SCT/(pMDI\ actuations/day)) \times total\ pMDI\ actuations$$

For a Ventolin™ HFA MDI, the total number of pMDI actuations is 200, and the number of pMDI actuations per day is 12. Thus, the AET is 2.5μg/pMDI closed container system.

The QT for any given pMDI (μg/pMDI closed container system) can be determined as follows:

$$QT = (Daily\ QT/actuations/day)) \times total\ pMDI\ actuations$$

The QT for Ventolin™ HFA MDI is therefore 83.3 μg/pMDI closed container system

The observed level of 2.7 mg/pMDI closed container system for PEG 4000 leachable from desiccant plastic blend X1763 after 3 months at 40°C/75%RH does not fall within the AET and the QT, and therefore a toxicological safety assessment would likely be required if blend X1763 were to be used in Ventolin™ HFA MDI.

PEG 4000 Spiking Study

The purpose of this study was to understand whether the improvement in FPM stability in the MkA Des sub-batch was due to the desiccant-entrained material of the gathering ring 18 reducing the level of moisture in the suspension, or by the PEG 4000 leachable behaving similarly to a surfactant.

A batch of 100μg/actuation (200 actuation) Ventolin™ HFA MDIs fitted with Mk C valves (see Example 2) was manufactured. The formulation in three sub-batches was spiked with different levels of PEG 4000 and not spiked in another three control sub-batches (0A, 0D, 0N), as identified in Table 1. Table 1 also identifies the material of the gathering ring 18 in each sub-batch. Samples of each of these sub-batches were then stored at 30°C/65%RH and 40°C/75%RH and tested for FPM by ACI and for PEG leachables at initial and after 3 months of storage

**Table 1**

| Sub-batch | Spiked PEG (mg) | Gathering Ring Material |
|---|---|---|
| 1.0A | 1.0 | Acetal (unslotted) |
| 2.7A | 2.7 | Acetal (unslotted) |
| 5.0A | 5.0 | Acetal (unslotted) |
| 0A | 0.0 | Acetal (unslotted) |
| 0D | 0.0 | X1763 Desiccant (unslotted) |
| 0N | 0.0 | Nylon (slotted) |

No difference was detected in performance between the sub-batches with the three different levels of spiked PEG 4000, and also these sub-batches exhibited a decrease in FPM after 3 months storage. From this it may be concluded that the PEG 4000 leachable from the X1763 blend ring is not responsible for the observed stabilisation in FPM in the MkA Des sub-batch, but the result of moisture uptake by the X1763 blend in the gathering ring 18. Figure 4A summarises the PEG 4000 leachables results after storage at two different storage conditions for the control sub-batches with Mk C valves having a simple, solid (unslotted) ring 18 made either of acetal (OA) or the X1763 blend (0D). At both conditions, no PEG 4000 is present in the 0A sub-batch, as expected, whilst for the X1763 sub-batch (0D), PEG 4000 is present as a leachable at initial (-50 μg/inhaler) and increases after 3 months storage, particularly at 40°C/75%RH, where over 3.5mg/pMDI closed container system is detected. This indicates that temperature and/or humidity greatly influence the level of PEG 4000 which leaches and enters the suspension.

## Example 2

In Example 2, the components of the metering valve assembly of the pMDI closed container system batch were (with the exception of control sub-batch C *infra*) made of the following materials:

| Component (ref. no. in Fig. 2) | Material |
|---|---|
| Valve body (1) | Acetal (two orifices 26) |

(continued)

| Component (ref. no. in Fig. 2) | Material |
|---|---|
| Spring (6) | Stainless steel |
| Valve stem, upper+lower parts (8,8a) | Acetal |
| Ferrule (2) | Aluminium |
| Sleeve (14) | Acetal |
| Stem seals (9, 12) | Nitrile rubber |
| Gasket (3) | Nitrile rubber |
| Ring (18) | Nylon (six slots 18c) |

This is hereinafter referred to as the "Mark B" or "Mk B" valve. The Mk B valve has a valve body 1 with orifices 26 as shown in Figure 2, not slots as in the Mk A valve used in Example 1.

Table 2 gives details of the sub-batches for Example 2. Prior to filling, into the canister of each sub-batch (other than control sub-batches B and C) there was inserted a lozenge made from the materials given in Table 2.

In more detail, the lozenge of each X series sub-batch was of a solidified blend composition of a channelling agent, base resin and desiccant in the proportions shown. In other words, like the gathering ring 18 in the Mk A Des sub-batch in Example 1, the X series sub-batches in Example 2 contained a lozenge made from a desiccant-entrained material of the type defined and available from CSP Technologies (Auburn, Alabama, USA).

As controls, the sub-batches NC, MC and PC were provided with lozenges made from nylon (NC) or polypropylene (MC and PC). The MC control used a Metocene™ grade of polypropylene, while the PC control used a Purell™ pharmaceutical grade of polypropylene. As will be gathered, these controls used lozenges of the different base resins in the X series sub-batches.

As a further controls, sub-batches B and C did not contain any lozenge. The B sub-batch used the Mark B valve, like the X series, NC, MC and PC sub-batches. However, the C sub-batch used a valve which corresponded to the Mark B valve, other than that the gasket 3 was made of ethylene-propylene diene monomer (EPDM) rubber. This is hereinafter referred to as the "Mark C" or "Mk C" valve.

### Table 2

| | LOZENGE COMPOSITION | | | | | |
|---|---|---|---|---|---|---|
| Sub-batch Number | Channelling Agent (average molecular weight) | % (by mass) | Base Resin | % (by mass) | Desiccant | % (by mass) |
| X1966 | PEG 20000^ (16000-24000) | 1 | Nylon* | 59 | Ceca 4A molecular sieve | 40 |
| X1967 | PEG 20000^ (16000-24000) | 3 | Nylon* | 57 | Ceca 4A molecular sieve | 40 |
| X1968 | PEG 20000^ (16000-24000) | 1 | Polypropylene** (Metocene™) | 59 | Ceca 4A molecular sieve | 40 |
| X1969 | PEG 20000^ (16000-24000) | 3 | Polypropylene** (Metocene™) | 57 | Ceca 4A molecular sieve | 40 |
| X1970 | PEG 20000^ (16000-24000) | 1 | Polypropylene*** (Purell™) | 59 | Ceca 4A molecular sieve | 40 |
| X1971 | PEG 20000^ (16000-24000) | 3 | Polypropylene*** (Purell™) | 57 | Ceca 4A molecular sieve | 40 |

(continued)

| Sub-batch Number | Channelling Agent (average molecular weight) | % (by mass) | Base Resin | % (by mass) | Desiccant | % (by mass) |
|---|---|---|---|---|---|---|
| X1972 | EVA | 1 | Polypropylene*** (Purell™) | 59 | Ceca 4A molecular sieve | 40 |
| X1973 | EVA | 3 | Polypropylene*** (Purell™) | 57 | Ceca 4A molecular sieve | 40 |
| NC | | | Nylon* | 100 | | |
| MC | | | Polypropylene** (Metocene™) | 100 | | |
| PC | | | Polypropylene*** (Purell™) | 100 | | |
| Control B (No lozenge) | | | | | | |
| Control C (No lozenge) | | | | | | |

Key: ^ polyethylene glycol (polyglykol 20000S, Clariant)
EVA = Ethylene Vinyl Acetate (Elvax™ 880, E. I. Du Pont de Nemours and Company) * Zytel™ 101, E. I. Du Pont de Nemours and Company, USA.
** PP = Metocene™ HM560P, LyondellBasell.
***PP = Purell™ HP570R, LyondellBasell.

A number of closed container systems in each sub-batch were stored inverted at 30°C/65%RH and 40°C/75%RH for up to 12 months. At different time points in the storage period five closed container systems from each sub-batch at the different storage conditions were removed and tested for FPM and moisture level. The FPM was tested according to the US standard, meaning the FPM is the sum of stages 3-5 inclusive of the ACI.

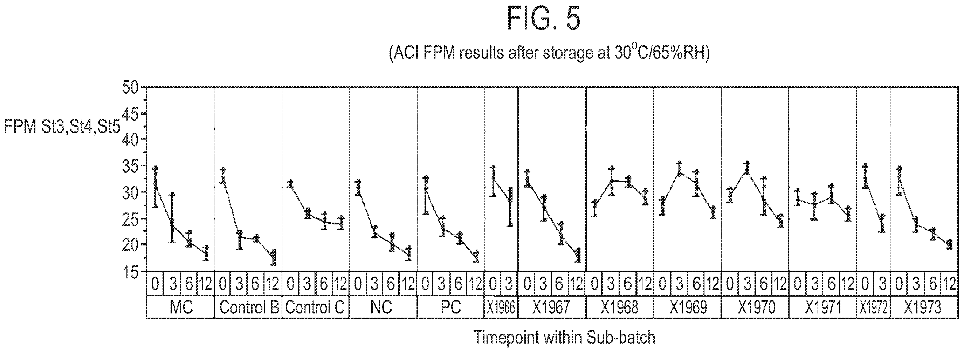

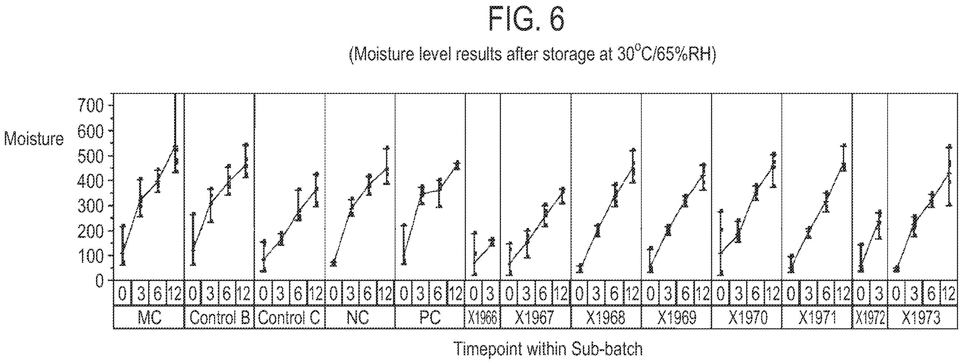

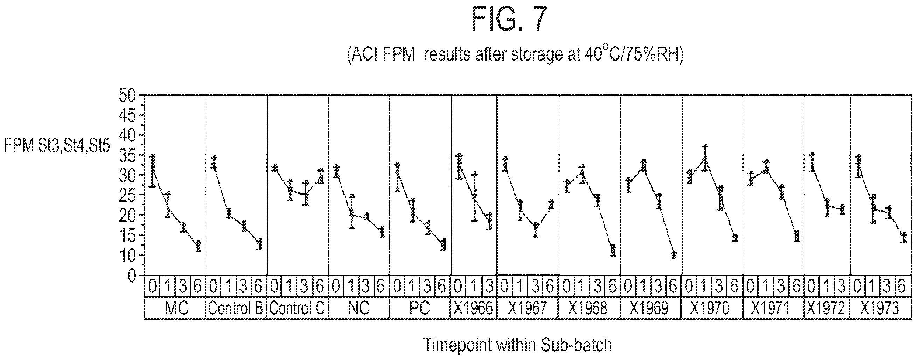

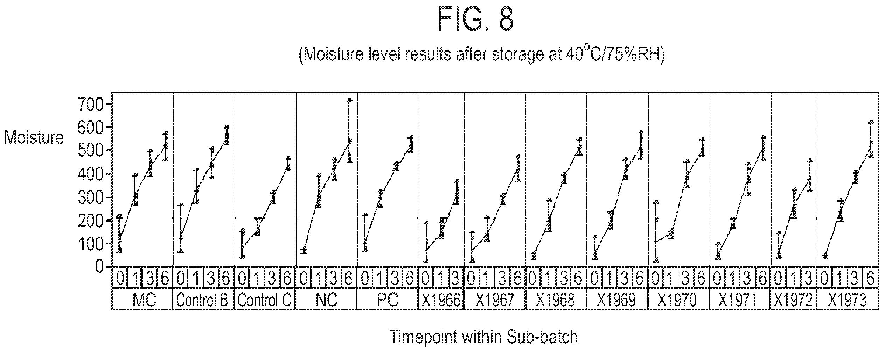

The results at 30°C/65%RH are shown in Figures 5 and 6 and at 40°C/75%RH in Figures 7 and 8.

From Figures 5 and 6, it can be seen that for the X series sub-batches X1968-1971, and especially sub-batch X1971, the FPM decreased over the 12 month period much less than the controls despite continued moisture uptake by the suspension.

As sub-batches X1972 and X1973 corresponded to sub-batches X1970 and X1971 other than the channelling agent, the results suggest that PEG is a better channelling agent than EVA. Likewise, the results suggest that polypropylene is better as the base resin than nylon as this was the only difference between sub-batches X1966/X1967 and sub-batches X1970/X1971.

From Figures 7 and 8 it can be seen that at the harsher storage condition of 40°C/75%RH the FPM drops off after 6 months for all sub-batches tested, except the control sub-batch C which exhibited a rebound after 3 months. The level of moisture in the suspension increased in all sub-batches over the 6 month storage period at 40°C/75 %RH.

The results of Example 2 show that blends X1968 to X1971, all of which are blend variations of polypropylene, PEG with an average molecular weight in the range of 16000-24000 (PEG 20000) and Ceca 4A molecular sieve, offer an improvement to FPM stability at 30°C/65%RH, despite an increase in suspension moisture level. Sub-batch X1971 appears to have performed best. Moreover, the different grades of polypropylene base polymer produce comparable product performance results. The FPM stability in blends X1968 to X1971 is unlikely due to a polypropylene leachable, as the FPM decreases in the MC and PC control sub-batches containing the polypropylene control lozenges.

**Measurement of PEG 20000 as a Leachable**

All of the X series sub-batches of Example 2, other than X1972 and X1973, used PEG 20000 as the channelling agent, rather than PEG 4000 used in the X1763 blend of Example 1. It was hypothesised that PEG 20000 may leach from the desiccant plastic lozenge into the formulation at a lower rate due to the higher molecular weight. In order to assess PEG 20000 as a potential leachable, an LC-MS method was developed.

The PEG 4000 measurement method was used as a starting point for the development of an LC(ESI)-MS method to detect PEG 20000. Similar difficulties existed in using LC-MS with PEG 20000 as did for PEG 4000. Ionisation was achieved with ESI, but the multiple charging was much greater than previously observed for PEG 4000, and therefore it was not possible to select ions for SIM analysis. Instead, the method was optimised to detect PEG 20000 in the positive m/z scan range of 700 to 1200. An experimental limit test for PEG 20000 was developed and validated. The limit was set as 62.5 μg/inhaler, which is 20.8 μg/inhaler lower than the QT for Ventolin™ HFA MDI (see Example 1).

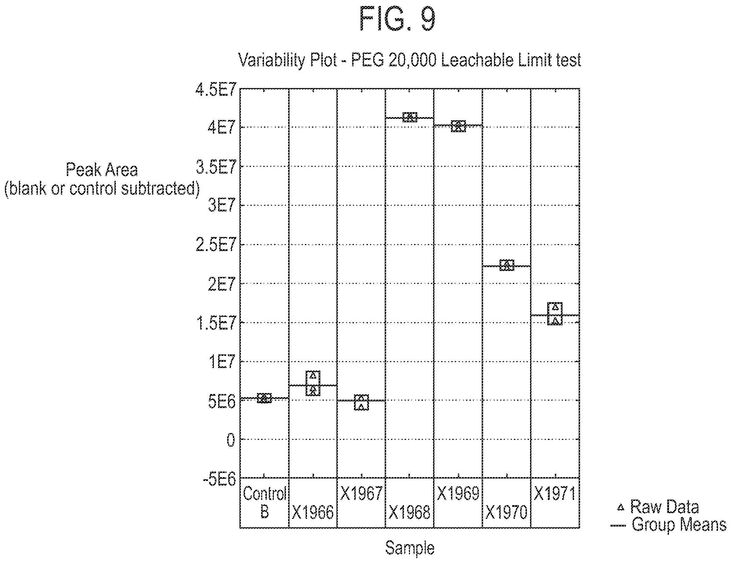

A batch of placebo (200 actuation) closed container systems were made up of sub-batches containing the six different lozenge blends of this Example which contained PEG 20000 as the channelling agent, and one sub-batch of Control B which contained no lozenge. These were stored for 3 months at 40°C/75%RH. Following storage, the samples were analysed for the presence of PEG 20000 as a leachable (see Figure 9; Y axis is the peak area (area under the line in chromatography) that determines the amount of the leachable). The data show that sub-batches X1966, X1967, X1970 and X1971 had PEG 20000 levels of less than 62.5 μg/inhaler, whilst sub-batches X1968 and X1969 had greater than 62.5 μg/inhaler. By increasing the average molecular weight of the PEG by changing from PEG 4000 to PEG 20000 reduced the PEG leachable level from -2.7 mg/inhaler to -50 μg/inhaler. Thus, the levels of PEG 20000 as a leachable are significantly lower than the level of PEG 4000 leachable, meaning a toxicology assessment would be less likely required for PEG 20000 (i.e. PEG with an average molecular weight in the range of 16000-24000). It can also be hypothesised that this would be the case for PEG with even higher average molecular weights.

## Example 3

Example 3 was carried out with the sub-batches referred to in Table 3. The X series, AC and PC sub-batches each comprised 50 closed container systems utilising valves corresponding to the Mk B valves used in Example 2, other than the gathering ring 18 being made from different desiccant-entrained and control plastics materials as identified in Table 3. The X1763 sub-batch used a ring made from the X1763 blend of Example 1. The X1971 sub-batch corresponded to the same sub-batch of Example 2. The X2010 and X2011 sub-batches used a desiccant-entrained material of the type defined (CSP Technologies, Auburn, Alabama, USA) having the same materials as X1971, but in different mass ratios. The X2012 and X2013 sub-batches used a desiccant-entrained material of the type defined (CSP Technologies, Auburn, Alabama, USA) having the same polypropylene and molecular sieve materials as the other X series sub-batches, but the channelling agent was PEG of a much higher average molecular weight. The rings 18 for the X series sub-batches and the AC and PC control sub-batches were not slotted, but simple solid ring forms. Two further control sub-batches were also tested, one with the Mk B valve (Control B sub-batch) and the other with the Mk C valve (Control C sub-batch). The Control B and C sub-batches both had a slotted gathering ring 18 (six slots 18c) made from nylon.

**Table 3**

| Sub-batch Number | Channeling Agent (average molecular weight) | % (by mass) | Base Resin | % (by mass) | Desiccant | % (by mass) |
|---|---|---|---|---|---|---|
| | **MATERIAL FOR VALVE RING** | | | | | |
| X1763 | PEG 4000* (3600-4400) | 5 | Acetal | 35 | Ceca 4A molecular sieve | 60 |
| X1971 | PEG 20000** (16000-24000) | 3 | Polypropylene^ (Purell™) | 57 | Ceca 4A molecular sieve | 40 |
| X2010 | PEG 20000** (16000-24000) | 3 | Polypropylene^ (Purell™) | 47 | Ceca 4A molecular sieve | 50 |

(continued)

| Sub-batch Number | MATERIAL FOR VALVE RING | | | | | |
| | Channeling Agent (average molecular weight) | % (by mass) | Base Resin | % (by mass) | Desiccant | % (by mass) |
|---|---|---|---|---|---|---|
| X2011 | PEG 20000** (16000-24000) | 10 | Polypropylene^ (Purell™) | 40 | Ceca 4A molecular sieve | 50 |
| X2012 | PEG 300000*** (270000-330000) | 3 | Polypropylene^ (Purell™) | 47 | Ceca 4A molecular sieve | 50 |
| X2013 | PEG 300000*** (270000-330000) | 10 | Polypropylene^ (Purell™) | 40 | Ceca 4A molecular sieve | 50 |
| AC (acetal control) | - | - | Acetal ^^ | 100 | - | - |
| PC (polypropylene control) | - | - | Polypropylene^ (Purell™) | 100 | - | - |
| Control B | - | - | - | - | - | - |
| Control C | - | - | - | - | - | - |

* Carbowax 4000P, The Dow Chemical Company
*** PolyOx 750, The Dow Chemical Company
^^ Ticona Celcon
** polyglykol 20000S, Clariant
^ Purell™ HP570R, LyondellBasell.

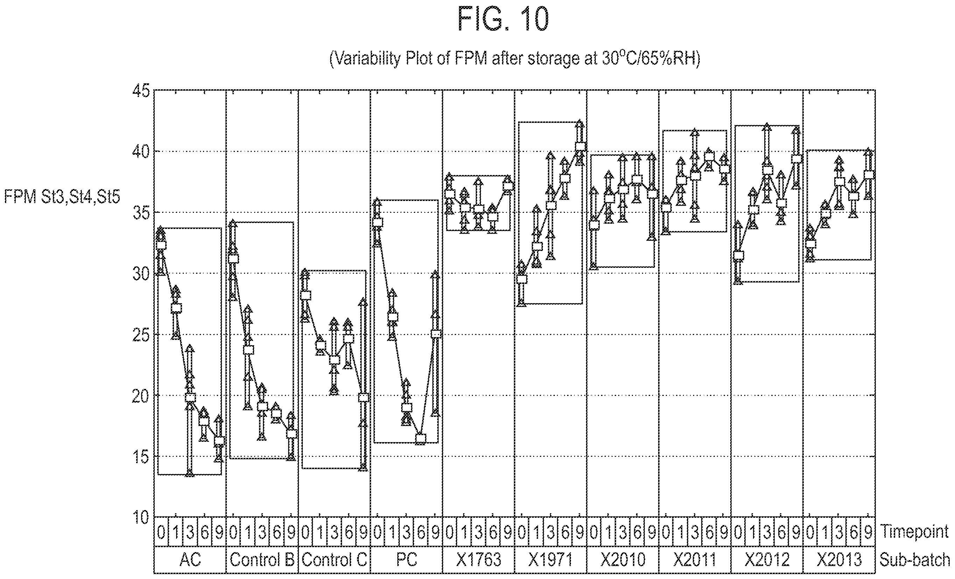

Samples from each sub-batch were stored inverted and unprotected either at 30°C/65%RH or at 40°C/75%RH for up to 9 months. At different time points in the storage period a number of closed container systems from each sub-batch were removed and tested for FPM and moisture level. At the 0 and 3 month time points, five samples from each sub-batch were tested, and three samples tested at the other time points. The results are shown in Figures 10-15.

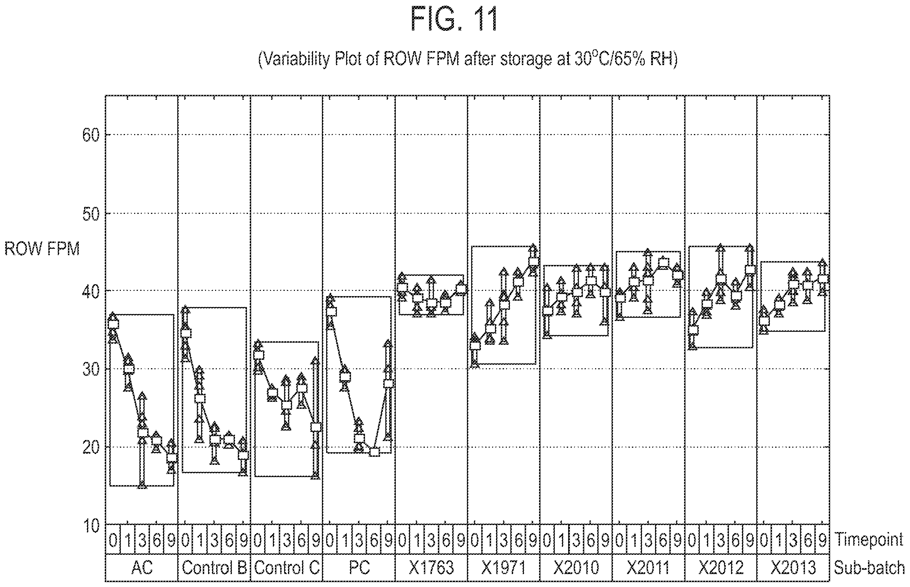

As respectively shown in Figures 10 and 11, the FPM results according to the US and RoW standards for the X series sub-batches over 9 months at 30C/65%RH are consistent compared to the controls.

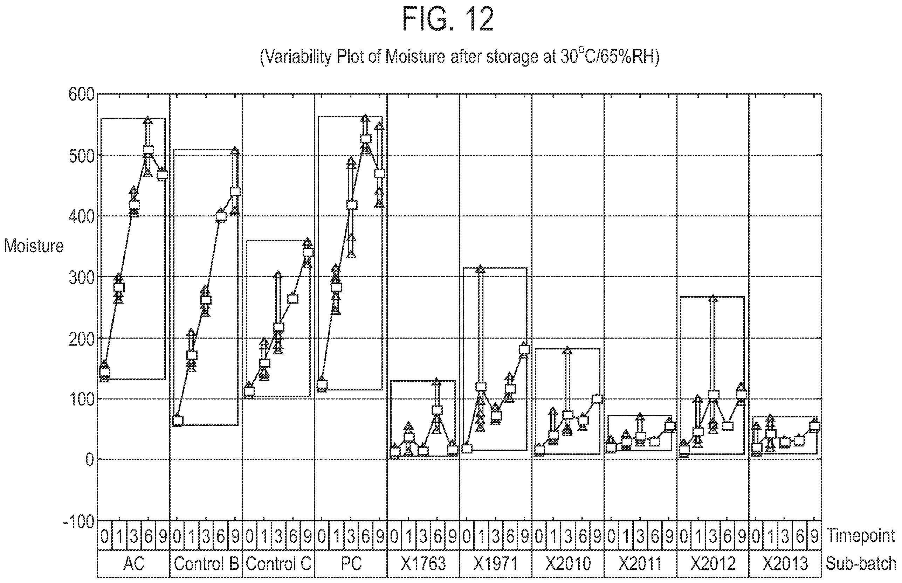

As shown in Figure 12, the level of moisture in the X series sub-batches is also markedly less than the controls for the 9 month period at 30C/65%RH. The improved control of the moisture level in the X series sub-batches compared to the controls is thought to give rise to the improved stability of the FPM of the X series sub-batches. Without wishing to be bound by theory, one explanation is that an increased level of moisture leads to agglomeration of drug particles to a size with an aerodynamic diameter too large to be included in the FPM.

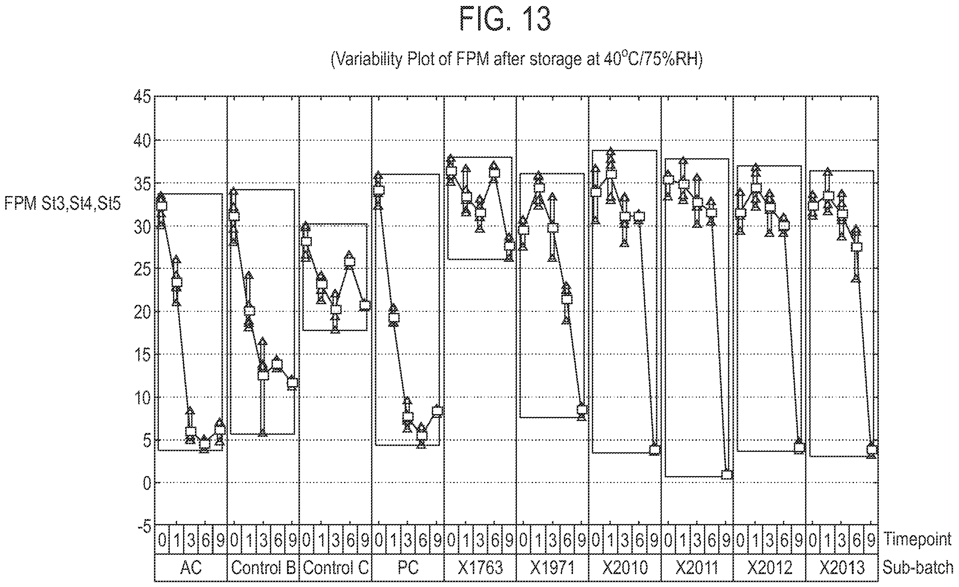

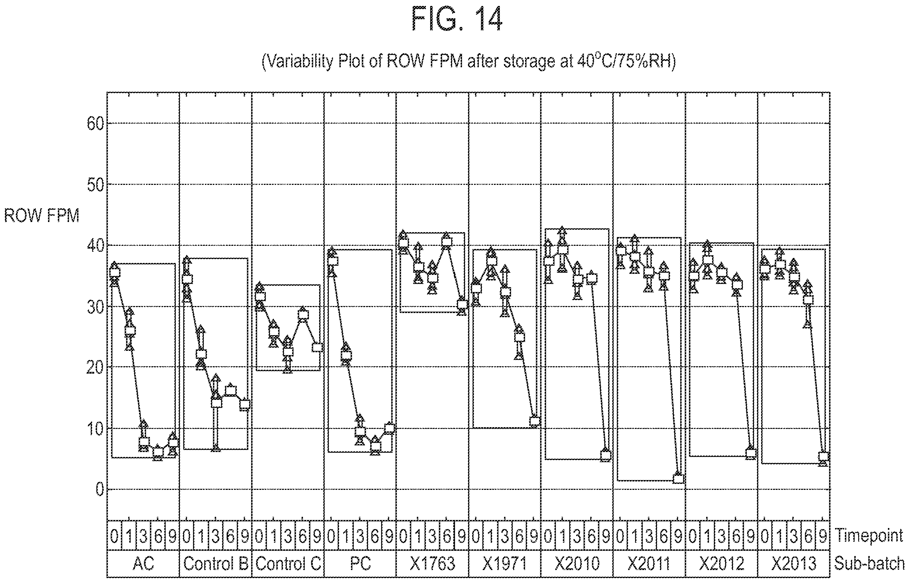

Figures 13 and 14 respectively show the US and RoW FPM data collected at time points up to 9 months for the sub-batches stored at 40°C/75% RH.

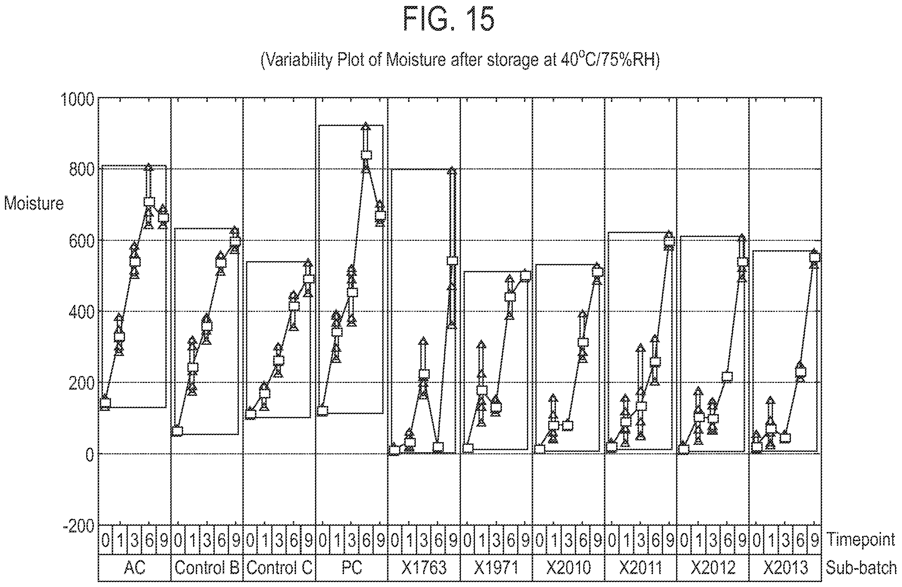

As will be observed, the FPM is consistent for all X series sub-batches up to 6 months, except for X1971. At 9 months there is a marked drop-off in the FPM for all of the X series sub-batches, except X1763. From Figure 15, this appears to correspond to a marked increase in the moisture levels in the X series sub-batches after 6 months storage. However, as previously described above, as the X series sub-batches produce stable FPM for 6 months at 40°C/75%RH, this represents an 18 month shelf-life for these sub-batches at the normal stability condition for the FDA.

An interpretation of this accelerated condition data is that the desiccant in the valve ring 18 of the X series sub-batches other than X1763 reached the saturation point by 9 months, with saturation occurring before 6 months for the X1971 sub-batch and between 6 and 9 months for the X2010-2013 sub-batches. Once saturated, the desiccant was unable to continue to adsorb moisture in the closed container system. This may be attributable to the reduced amount of molecular sieve in these X series sub-batches compared to X1763. It may therefore be advantageous to have the desiccant in an amount greater than 40%, optionally up to about 60% by mass (by mass of the overall plastics composition, i.e. as in the X2010-2013 and X1763 sub-batches.

Sub-batches X2012 and X2013 of Example 3 demonstrate that the stabilising effect of the desiccant-entrained material on the FPM is also achieved with PEG as the channelling agent at a much higher average molecular weight.

The Examples show that a more consistent FPM can be attained through use of a desiccant-entrained material of the type defined in the closed container system to contact the inhalation drug formulation, whether comprised in a loose component or a valve component. The Examples further show that good results are obtained with PEG as the channelling agent for a wide range of average molecular weights up to PEG 300000. However, Examples 1 and 2 demonstrate that PEG 4000 leaches more than higher average molecular weight PEGs and so it may be preferable to use PEG greater than PEG 4000, especially since this is demonstrated to not adversely affect the stabilising effect of the desiccant-entrained material on FPM.

With regard to Example 2, it is believed that improved results would be achieved with a lozenge or similar such loose component in the canister if the composition of the desiccant-entrained material was denser that the density of the inhalation drug formulation so as to maximise the outer surface in contact with the formulation, for instance so as to be immersed in the formulation so that all outer surfaces are in contact with the formulation.

The present invention is not limited to the preceding Examples, but embraces all variants within the full scope of the appended claims.

The use of relative terms such as "about" and the like in relation to a structural or numerical parameter is to be taken as including the exact structural or numerical parameter and also minor deviations therefrom.

## Claims

1. Use of a desiccant-entrained material of the type defined in a pMDI closed container system to stabilise the fine particle mass (FPM) of an inhalation drug formulation emitted by the system, wherein the desiccant-entrained material is a mixture of a (i) base polymer, (ii) desiccant and (iii) a channelling agent that facilitates the transmission of water into the material, wherein the channelling agent is polyethylene glycol (PEG) of an average molecular weight of at least 8000.

2. Use according to claim 1 in which the desiccant-entrained material is able to stabilise the FPM without use of a moisture impermeable overwrap.

3. Use according to any preceding claim resulting in the system having a stable FPM after storage for a period of at least 6 months at a temperature of 40°C and 75% relative humidity (RH).

4. Use according to any preceding claim, wherein the desiccant-entrained material is comprised in a metering valve of the closed container system; and/or wherein the desiccant-entrained material is comprised in a component of the closed container system which is in contact with the inhalation drug formulation, said component optionally being loose inside the canister.

5. Use according to any preceding claim, wherein the channelling agent polymer has an average molecular weight of at least 16000, and optionally no more than 350000.

6. Use according to any preceding claim, wherein the channelling agent is present in an amount of up to 15% by mass of the desiccant-entrained material, optionally in the range of 1-10% by mass and further optionally in the range of 3-10% by mass.

7. Use according to any preceding claim, wherein:

   the base polymer is a thermoplastics polymer, for example polypropylene; and/or
   the base polymer is present in the range of 30-70% by mass of the desiccant-entrained material, optionally in the range of 35-60% by mass, yet further optionally in the range of 40-60% by mass and more optionally in the range of 40-50% by mass.

8. Use according to any preceding claim, wherein:

   the desiccant is a molecular sieve, for example having a pore size of 4 Angstroms; and/or
   the desiccant is present in the range of 30-70% by mass of the desiccant-entrained material, optionally in the range of 40-60% by mass, more optionally in the range of 45-60% by mass, further optionally in the range of 45-55% by mass, yet further optionally in the range of 45-50% by mass; and/or
   the desiccant-entrained material comprises the base polymer in the range of 35-60% by mass, optionally in the range of 40-60%, the desiccant in the range of 40-60% by mass, optionally in the range of 45-60% by mass, and the channelling agent polymer in the range of 1-15% by mass, optionally 3-10% by mass.

9. Use according to any of claims 1 to 8, wherein the desiccant-entrained material is a blend of polypropylene, PEG as the channelling agent, and a molecular sieve, optionally with a pore size of 4 Angstroms.

10. Use according to any preceding claim, wherein the formulation comprises salbutamol or a physiologically acceptable salt thereof, and optionally the formulation comprises salbutamol sulphate and a CFC-free propellant.

11. Use according to claim 10, wherein the drug formulation comprises salbutamol sulphate and a CFC-free propellant and optionally wherein the PEG is of an average molecular weight of at least 16000.

12. A pMDI metering valve comprising at least one component which comprises a desiccant-entrained material of the type defined which is a mixture of a (i) base polymer, (ii) desiccant and (iii) a channelling agent that facilitates the transmission of water into the material, wherein the channelling agent is polyethylene glycol (PEG) of an average molecular weight of at least 8000.

13. A pMDI closed container system containing a desiccant-entrained material of the type defined which is a mixture of a (i) base polymer, (ii) desiccant and (iii) a channelling agent that facilitates the transmission of water into the material, wherein the channelling agent is polyethylene glycol (PEG) of an average molecular weight of at least 8000.

14. A method of stabilising the fine particle mass (FPM) of an inhalation drug formulation emitted by a pMDI closed container system by providing a desiccant-entrained material of the type defined inside the pMDI closed container system so as to be in contact with the formulation therein, and optionally wherein the FPM is stable after storage of the system for a period of at least 6 months at a temperature of 40°C and 75% relative humidity (RH) as determined with an Anderson cascade impactor, and wherein the desiccant-entrained material is a mixture of a (i) base polymer, (ii) desiccant and (iii) a channelling agent that facilitates the transmission of water into the material, wherein the channelling agent is polyethylene glycol (PEG) of an average molecular weight of at least 8000.

**Patentansprüche**

1. Verwendung von einem mit Trocknungsmittel versetzten Materials von dem definierten Typ in einem pMDI geschlossenen Behältersystem, um die Feinpartikelmasse (FPM) einer inhalierbaren Arzneimittelformulierung emittiert von dem System zu stabilisieren, wobei das mit Trocknungsmittel versetzte Material eine Mischung von einem (i) Grundpolymer, (ii) Trocknungsmittel und (iii) einem Kanalbildungsmittel, welches die Übertragung von Wasser in das Material fördert, wobei das Kanalbildungsmittel Polyethylenglykol (PEG) mit einem durchschnittlichen Molekulargewicht von mindestens 8000 ist.

2. Verwendung gemäß Anspruch 1, in welcher das mit Trocknungsmittel versetzte Material die FPM ohne Verwendung von einer feuchtigkeitsundurchlässsigen Umhüllung stabilisieren kann.

3. Verwendung gemäß einem der vorhergehenden Ansprüche, welche in einem System mit stabiler FPM nach Lagerung über einen Zeitraum von mindestens 6 Monaten bei einer Temperatur von 40°C und 75% relative Feuchtigkeit (RH) resultiert.

4. Verwendung gemäß einem der vorhergehenden Ansprüche, wobei das mit Trocknungsmittel versetzte Material in einem Dosierventil des geschlossenen Behältersystems enthalten ist; und/oder wobei das mit Trocknungsmittel versetzte Material in einer Komponente des geschlossenen Behältersystems, welches in Kontakt mit der inhalierbaren Arzneimittelformulierung ist, enthalten ist, wobei die genannte Komponente gegebenenfalls lose innerhalb des Kanisters vorliegt.

5. Verwendung gemäß einem der vorhergehenden Ansprüche, wobei das Kanalbildungsmittelpolymer ein durchschnittliches Molekulargewicht von mindestens 16000, und gegebenenfalls nicht mehr als 350000 hat.

6. Verwendung gemäß einem der vorhergehenden Ansprüche, wobei das Kanalbildungsmittel in einer Menge von bis zu 15 Massen-% des mit Trocknungsmittel versetzten Materials, gegebenenfalls in einem Bereich von 1-10 Massen-% und weiter gegebenenfalls in einem Bereich von 3-10 Massen-% vorhanden ist.

7. Verwendung gemäß einem der vorhergehenden Ansprüche, wobei:

das Grundpolymer ein thermoplastisches Polymer, zum Beispiel Polypropylen ist; und/oder
das Grundpolymer in einem Bereich von 30-70 Massen-% des mit Trockenmittel versetzten Material, gegebenenfalls in einem Bereich von 35-60 Massen-%, noch weiter gegebenenfalls in einem Bereich von 40-60 Massen-% und mehr gegebenenfalls in einem Bereich von 40-50 Massen-%, vorhanden ist.

8. Verwendung gemäß einem der vorhergehenden Ansprüche, wobei:

das Trocknungsmittel ein Molekularsieb, zum Beispiel mit einer Porengröße von 4 Ångström, ist; und/oder
das Trocknungsmittel in einem Bereich von 30-70 Massen-% des mit Trocknungsmittel versetzten Materials, gegebenenfalls in einem Bereich von 40-60 Massen-%, mehr gegebenenfalls in einem Bereich von 45-60 Massen-%, weiter gegebenenfalls in einem Bereich von 45-55 Massen-%, noch weiter gegebenenfalls in einem Bereich von 45-50 Massen-%, vorhanden ist; und /oder
das mit Trocknungsmittel versetzte Material das Grundpolymer in einem Bereich von 35-60 Massen-%, gegebenenfalls in einem Bereich von 40-60%, das Trocknungsmittel in einem Bereich von 40-60 Massen-%, gegebenenfalls in einem Bereich von 45-60 Massen-%, und das Kanalbildungsmittelpolymer in einem Bereich von 1-15 Massen-%, gegebenenfalls 3-10 Massen-%, umfasst.

9. Verwendung gemäß einem der Ansprüche 1 bis 8, wobei das mit Trocknungsmittel versetzte Material eine Mischung von Polypropylen, PEG als das Kanalbildungsmittel, und ein Molekularsieb, gegebenenfalls mit einer Porengröße von 4 Ängström ist.

10. Verwendung gemäß einem der vorhergehenden Ansprüche, wobei die Formulierung Salbutamol oder ein physiologisch verträgliches Salz davon umfasst, und gegebenenfalls die Formulierung Salbutamolsulfat und ein CFC-freies Treibmittel umfasst.

11. Verwendung gemäß Anspruch 10, wobei die Arzneimittelformulierung Salbutamolsulfat und ein CFC-freies Treibmittel und gegebenenfalls das PEG ein Molekulargewicht von mindestens 16000 aufweist, umfasst.

12. Ein pMDI Dosierventil umfassend mindestens eine Komponente, welche ein mit Trocknungsmittel versetztes Material von dem definierten Typ, welches eine Mischung von einem (i) Grundpolymer, (ii) Trocknungsmittel und (iii) einem Kanalbildungsmittel, welches die Übertragung von Wasser in das Material fördert, wobei das Kanalbildungsmittel Polyethylenglykol (PEG) mit einem durchschnittlichen Molekulargewicht von mindestens 8000 ist, umfasst.

13. Ein pMDI geschlossenes Behältersystem enthaltend ein mit Trocknungsmittel versetztes Material von definiertem Typ, welches eine Mischung von einem (i) Grundpolymer, (ii) Trocknungsmittel, und (iii) einem Kanalbildungsmittel, welches die Übertragung von Wasser in das Material fördert, wobei das Kanalbildungsmittel Polyethylenglykol (PEG) mit einem durchschnittlichen Molekulargewicht von mindestens 8000 ist.

14. Ein Verfahren zur Stabilisierung der Feinpartikelmasse (FPM) einer inhalierbaren Arzneimittelformulierung, emittiert von einem pMDI geschlossenen Behältersystem, durch Bereitstellung eines mit Trocknungsmittel versetzten Materials von dem definierten Typ innerhalb des pMDI geschlossenen Behältersystems, um mit der Formulierung darin in Kontakt zu sein, und gegebenenfalls wobei die FPM nach Lagerung des Systems über einen Zeitraum von mindestens 6 Monaten bei einer Temperatur von 40°C und 75% relativer Feuchtigkeit (RH), wie mit einem Anderson Kaskadenimpaktor bestimmt, stabil ist, und wobei das mit Trocknungsmittel versetzte Material eine Mischung von einem (i) Grundpolymer, (ii) Trocknungsmittel und (iii) einem Kanalbildungsmittel, welches die Übertragung von Wasser in das Material fördert, wobei das Kanalbildungsmittel Polyethylenglykol (PEG) mit einem durchschnittlichen Molekulargewicht von mindestens 8000 ist.

## Revendications

1. Utilisation d'un matériau à déshydratant intégré du type défini dans un système de récipient fermé pMDI pour stabiliser la masse de fines particules (FPM) d'une formulation médicamenteuse d'inhalation émise par le système, dans laquelle le matériau à déshydratant intégré est un mélange (i) d'un polymère de base, (ii) d'un déshydratant et (iii) d'un agent de canalisation qui facilite la transmission d'eau dans le matériau, dans laquelle l'agent de canalisation est le polyéthylèneglycol (PEG) d'un poids moléculaire moyen d'au moins 8 000.

**2.** Utilisation selon la revendication 1, dans laquelle le matériau à déshydratant intégré est à même de stabiliser la FPM sans utiliser une enveloppe imperméable à l'humidité.

**3.** Utilisation selon l'une quelconque des revendications précédentes entraînant dans le système le fait qu'il ait une FPM stable après stockage pendant une période d'au moins 6 mois à une température de 40 °C et 75 % d'humidité relative (RH).

**4.** Utilisation selon l'une quelconque des revendications précédentes, dans laquelle le matériau à déshydratant intégré est compris dans une vanne de mesure du système à récipient fermé ; et/ou dans laquelle le matériau à déshydratant intégré est compris dans un composant du système de récipient fermé qui est en contact avec la formulation médicamenteuse d'inhalation, ledit composant étant éventuellement libre à l'intérieur de la cannette.

**5.** Utilisation selon l'une quelconque des revendications précédentes, dans laquelle le polymère de l'agent de canalisation a un poids moléculaire d'au moins 16 000 et éventuellement de pas plus de 350 000.

**6.** Utilisation selon l'une quelconque des revendications précédentes, dans laquelle l'agent de canalisation est présent en quantité allant jusqu'à 15 % en masse du matériau entraîné par le déshydratant, éventuellement dans la plage de 1 à 10 % en masse et encore éventuellement dans la plage de 3 à 10 % en masse.

**7.** Utilisation selon l'une quelconque des revendications précédentes, dans laquelle :

le polymère de base est un polymère thermoplastique, par exemple du polypropylène ; et/ou
le polymère de base est présent dans la plage de 30 à 70 % en masse du matériau à déshydratant intégré, éventuellement dans la plage de 35 à 60 % en masse, encore plus éventuellement dans la plage de 40 à 60 % en masse et mieux encore éventuellement dans la plage de 40 à 50 % en masse.

**8.** Utilisation selon l'une quelconque des revendications précédentes, dans laquelle :

le déshydratant est un tamis moléculaire, par exemple ayant une taille de pores de 4 angströms et/ou
le déshydratant est présent dans la plage de 30 à 70 % en masse du matériau à déshydratant intégré, éventuellement dans la plage de 40 à 60 % en masse, mieux encore éventuellement dans la plage de 45 à 60 % en masse, bien mieux encore éventuellement dans la plage de 45 à 55 % en masse, bien mieux encore éventuellement dans la plage de 45 à 50 % en masse ; et/ou
le matériau à déshydratant intégré comprend le polymère de base dans la plage de 35 à 60 % en masse, éventuellement dans la plage de 40 à 60 %, le déshydratant se situe dans la plage de 40 à 60 % en masse, éventuellement dans la plage de 45 à 60 % en masse, et le polymère d'agent de canalisation se situe dans la plage de 1 à 15 % en masse, éventuellement de 3 à 10 % en masse.

**9.** Utilisation selon l'une quelconque des revendications 1 à 8, dans laquelle le matériau à déshydratant intégré est un mélange de polypropylène PEG comme agent de canalisation et d'un tamis moléculaire éventuellement avec une taille de pores de 4 angströms.

**10.** Utilisation selon l'une quelconque des revendications précédentes, dans laquelle la formulation comprend du salbutamol ou un sel physiologiquement acceptable de celui-ci et éventuellement la formulation comprend du sulfate de salbutamol et un propulseur exempt de CFC.

**11.** Utilisation selon la revendication 10, dans laquelle la formulation médicamenteuse comprend du sulfate de salbutamol et un propulseur exempt de CFC et éventuellement dans laquelle le PEG a un poids moléculaire moyen d'au moins 16 000.

**12.** Vanne de mesure pMDI comprenant au moins un composant qui comprend un matériau à déshydratant intégré du type défini qui est un mélange (i) d'un polymère de base, (ii) d'un déshydratant et (iii) d'un agent de canalisation qui facilite la transmission de l'eau dans le matériau, dans laquelle l'agent de canalisation est le polyéthylèneglycol (PEG) d'un poids moléculaire moyen d'au moins 8 000.

**13.** Système de récipient fermé pMDI contenant un matériau à déshydratant intégré du type défini qui est un mélange (i) d'un polymère de base, (ii) d'un déshydratant et (iii) d'un agent de canalisation qui facilite la transmission de l'eau dans le matériau, dans lequel l'agent de canalisation est le polyéthylèneglycol (PEG) d'un poids moléculaire moyen d'au moins 8 000.

14. Procédé de stabilisation de la masse de fines particules (FPM) d'une formulation médicamenteuse d'inhalation émise par un système de récipient fermé pMDI en fournissant un matériau à déshydratant intégré du type défini à l'intérieur du système de récipient fermé pMDI de manière à être en contact avec la formulation qui s'y trouve et éventuellement dans lequel la FPM est stable après stockage du système pendant une période d'au moins 6 mois à une température de 40 °C et de 75 % d'humidité relative (RH) comme déterminé par un impacteur en cascade d'Anderson, et dans lequel le matériau à déshydratant intégré qui est un mélange (i) d'un polymère de base, (ii) d'un déshydratant et (iii) d'un agent de canalisation qui facilite la transmission de l'eau dans le matériau, dans lequel l'agent de canalisation est le polyéthylèneglycol (PEG) d'un poids moléculaire moyen d'au moins 8 000.

# FIG. 1

# FIG. 2

# FIG. 3

Valve Variant Study FPM and Moisture Results

FIG. 4

PEG 4000 Extractable Oligomer Series from HPLC-MS APCI Scan

EP 2 482 797 B1

## FIG. 4A

Variability Plot of PEG leachable (mg/can)

EP 2 482 797 B1

FIG. 5

(ACI FPM results after storage at 30°C/65%RH)

# FIG. 6

(Moisture level results after storage at 30°C/65%RH)

Timepoint within Sub-batch

## FIG. 7

(ACI FPM results after storage at 40°C/75%RH)

EP 2 482 797 B1

FIG. 8

(Moisture level results after storage at 40°C/75%RH)

Timepoint within Sub-batch

EP 2 482 797 B1

FIG. 9

Variability Plot - PEG 20,000 Leachable Limit test

Peak Area (blank or control subtracted)

Sample

△ Raw Data
— Group Means

FIG. 11

(Variability Plot of ROW FPM after storage at 30°C/65% RH)

FIG. 12

(Variability Plot of Moisture after storage at 30°C/65%RH)

EP 2 482 797 B1

# FIG. 13

(Variability Plot of FPM after storage at 40°C/75%RH)

## FIG. 14

(Variability Plot of ROW FPM after storage at 40°C/75%RH)

FIG. 15

(Variability Plot of Moisture after storage at 40°C/75%RH)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6131566 A
- WO 9942154 A
- US 6119853 A
- WO 0230498 A
- WO 0230499 A
- WO 03024623 A
- WO 9732663 A
- US 5911937 A
- US 6170717 B